# FASCICULE DE BREVET EUROPEEN

(11) **EP 2 516 661 B1**
(45) Date de publication et mention de la délivrance du brevet: **20.07.2016**
(21) Numéro de dépôt: 10808998.8
(22) Date de dépôt: 17.12.2010
(51) Int. Cl.: C12P 7/14, C12P 7/10, C12P 7/16, C12P 7/28

(54) **PROCÉDÉ DE PRODUCTION D'ALCOOLS ET/OU DE SOLVANTS À PARTIR DE PULPES PAPETIÈRES AVEC RECYCLAGE DU VÉGÉTAL NON HYDROLYSÉ DANS UN RÉACTEUR DE RÉGÉNÉRATION**
VERFAHREN ZUR HERSTELLUNG VON ALKOHOLEN UND/ODER LÖSUNGSMITTELN AUS FASERSTOFFEN MIT RECYCLING VON NICHT-HYDROLYSIERTEM PFLANZENMATERIAL IN EINEM REGENERATIONSREAKTOR
METHOD FOR PRODUCING ALCOHOLS AND/OR SOLVENTS FROM PAPER PULPS WITH RECYCLING OF THE NON-HYDROLYSATED PLANT MATERIAL IN A REGENERATION REACTOR

(30) Priorité: 23.12.2009 FR 0906334
(43) Date de publication de la demande: 31.10.2012
(73) Titulaire: IFP Énergies nouvelles, 92852 Rueil-Malmaison Cedex (FR)
(72) Inventeur: ROPARS, Marcel, F-91120 Palaiseau (FR); AYMARD, Caroline, F-69002 Lyon (FR); GUILLAUME, Anais, F-92500 Rueil Malmaison (FR)
(86) Numéro de dépôt international: PCT/FR2010/000851
(87) Numéro de publication internationale: WO 2011/086244

(56) Documents cités:
- EP-A2- 0 101 190
- WO-A1-2006/063467
- FR-A1- 2 923 840
- US-A1- 2009 093 027
- Hasegawa F et al.: "Bioethanol production from Kraft pulp", Sixth Biomass-Asia Workshop, 18 novembre 2009 (2009-11-18), XP2598882, Hiroshima, Japan Extrait de l'Internet: URL:http://www.biomass-asia-workshop.jp/bi omassws/06workshop/poster/P-33.pdf [extrait le 2010-08-31] -& Anonymous: "Sixth Biomas-Asia Workshop", , 2009, XP002598787, Hiroshima, Japan Extrait de l'Internet: URL:http://www.biomass-asia-workshop.jp/bi omassws/06workshop/ [extrait le 2010-08-31]
- MOONEY CAITRIONA A ET AL: "The effect of fiber characteristics on hydrolysis and cellulase accessibility to softwood substrates", ENZYME AND MICROBIAL TECHNOLOGY, vol. 25, no. 8-9, novembre 1999 (1999-11), pages 644-650, XP002598778, ISSN: 0141-0229
- MAIORELLA B L ET AL: "FEED COMPONENT INHIBITION IN ETHANOLIC FERMENTATION BY SACCHAROMYCES-CEREVISIAE", BIOTECHNOLOGY AND BIOENGINEERING, vol. 26, no. 10, 1984, pages 1155-1166, XP002598779, ISSN: 0006-3592

## Description

### DOMAINE DE L'INVENTION

La présente invention s'inscrit dans le cadre d'un procédé de production d'alcool et/ou de solvant dit de "seconde génération" à partir de biomasse lignocellulosique. Elle concerne plus particulièrement un procédé de production d'éthanol et/ou d'un mélange Acétone-Butanol-Ethanol (encore appelé mélange ABE).

### ART ANTÉRIEUR

La biomasse lignocellulosique représente une des ressources renouvelables les plus abondantes sur terre. Les substrats considérés sont très variés, puisqu'ils concernent à la fois les substrats ligneux (feuillus et résineux), les sous-produits de l'agriculture (paille) ou ceux des industries génératrices de déchets lignocellulosiques (industries agroalimentaires, papeteries).
La biomasse lignocellulosique est composée de trois principaux polymères : la cellulose (35 à 50%), l'hémicellulose (20 à 30%) qui est un polysaccharide essentiellement constitué de pentoses et d'hexoses et la lignine (15 à 25%) qui est un polymère de structure complexe et de haut poids moléculaire, composé d'alcools aromatiques reliés par des liaisons éther.

Ces différentes molécules sont responsables des propriétés intrinsèques de la paroi végétale et s'organisent en un enchevêtrement complexe.

La cellulose et éventuellement les hémicelluloses sont les cibles de l'hydrolyse enzymatique mais ne sont pas directement accessibles aux enzymes. C'est la raison pour laquelle ces substrats doivent subir un prétraitement précédant l'étape d'hydrolyse enzymatique. Le prétraitement vise à modifier les propriétés physiques et physico-chimiques du matériau lignocellulosique, en vue d'améliorer l'accessibilité de la cellulose emprisonnée au sein de la matrice de lignine et d'hémicellulose.
De nombreuses technologies pour réaliser ce prétraitement existent: cuissons acides, cuissons alcalines, explosion à la vapeur, procédés organosolv, etc. L'efficacité du prétraitement se mesure à la fois par le bilan matière à l'issue du prétraitement (taux de récupération des sucres sous forme monomère ou oligomère soluble ou polymères insolubles) et également par la susceptibilité à l'hydrolyse enzymatique des résidus cellulosiques et hémicellulosiques.

Les procédés de production d'alcools et/ou de solvants à partir de biomasse lignocellulosique, dit "procédés de seconde génération" comprennent au moins les étapes suivantes :
- prétraitement du substrat,
- hydrolyse enzymatique du substrat prétraité,
- fermentation de l'hydrolysat obtenu , et
- séparation /purification de l'alcool et/ou solvant obtenu après fermentation.

La validité économique de ce type de procédé de production d'alcool et/ou de solvant est difficile à obtenir même pour les opérateurs disposant d'une large ressource mobilisable. Deux postes ont un fort impact sur le coût global : la charge enzymatique nécessaire pour l'hydrolyse des sucres polymérisés et la matière végétale prétraitée. L'optimisation de ce type de procédé passe donc obligatoirement par une valorisation optimale de la charge enzymatique exprimée en kg de sucres libérés par kg ou FPu d'enzymes ajoutées. Ces conditions sont obtenues au moyen de faibles charges enzymatiques, typiquement de 5 à 10 g/kg de matière sèche. Malheureusement, ces faibles charges enzymatiques valorisent mal le substrat prétraité car le rendement d'hydrolyse est médiocre, notamment celui des glucanes qui constituent la cible essentielle car la conversion du glucose en éthanol et ABE est aisée.

La matière sèche insoluble soumise à l'hydrolyse enzymatique peut varier de 5 à 40%, et généralement entre 10 et 25%. D'après la publication de Kristensen et al. Biotechnology for biofuels, 2009 (2) 11, pour obtenir un rendement d'hydrolyse identique, la consommation enzymatique doit être plus élevée dans le cas d'une charge élevée de matière sèche insoluble, notamment en raison de l'inactivation de l'enzyme par les produits de l'hydrolyse enzymatique (glucose, cellobiose). La solution qui consisterait à réaliser une dilution au niveau de l'étape d'hydrolyse enzymatique est toutefois limitée puisqu'elle aura des conséquences importantes sur la dépense énergétique liée à la séparation de l'alcool réalisée par distillation. Dans le cas précis de la fabrication de l'éthanol, une concentration alcoolique du moût de fermentation avec 23-25g/L d'éthanol a minima (titre alcoolique de 3) est nécessaire pour une dépense raisonnable du poste de distillation.

D'autre part, pour optimiser ces procédés, il est souhaitable de maximiser la quantité de matière première hydrolysable et accessible aux enzymes, à partir d'une quantité initiale de biomasse.

En dehors des améliorations liées à l'efficacité des cocktails enzymatiques, l'amélioration du bilan économique de la production d'éthanol ou d'ABE peut être obtenu au moyen de recyclages de différents flux ou produits.

La demande de brevet WO 94/29475 propose un procédé de conversion amélioré de biomasse cellulosique en éthanol dans lequel une partie des effluents issus du fermenteur est recyclé à l'entrée du même fermenteur en tant que source de nutriments pour le micro-organisme utilisé lors de la fermentation.

La demande de brevet US 2009/0093027 décrit un procédé de fabrication de sucres et d'éthanol à partir de matières lignocellulosiques telles que le fourrage de maïs par l'addition de résidus de distillation obtenus dans un procédé de production d'éthanol à partir de graines de maïs.

Dans les procédés de seconde génération, pour améliorer leur rentabilité économique, on cherche à maximiser la quantité de sucres libérés, donc à maximiser le rendement, tout en maintenant une consommation de produits et d'enzymes la plus basse possible.

La présente invention décrit un procédé de production d'alcools et/ou de solvants amélioré adossé à une unité de production de pâtes papetières utilisant un prétraitement chimique alcalin.

### RÉSUMÉ DE L'INVENTION

La présente invention porte sur un procédé de production d'alcools et/ou de solvants dit de seconde génération, dans lequel la biomasse lignocellulosique ou cellulosique subit un prétraitement alcalin puis un recyclage des pâtes non hydrolysées enzymatiquement est opéré, après passage desdites pâtes dans un réacteur de régénération de la cellulose.

### DESCRIPTION DES DESSINS

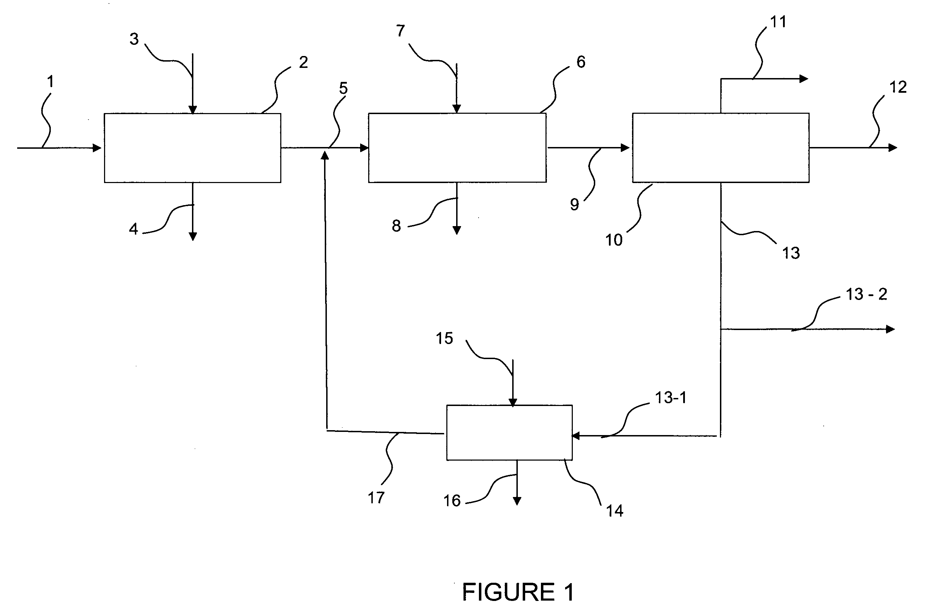

La Figure 1 est une représentation schématique d'un dispositif mettant en oeuvre un procédé de production d'alcools et/ou de solvants à partir de pulpes papetières, comprenant une étape de recyclage des résidus solides, selon la présente invention.

### DESCRIPTION DÉTAILLÉE DE L'INVENTION

La présente invention décrit un procédé de production d'alcools et/ou de solvants à partir de biomasse cellulosique ou lignocellulosique comprenant au moins les étapes suivantes :
a) prétraitement chimique alcalin d'un substrat cellulosique ou lignocellulosique ;
b) éventuellement lavage du substrat prétraité ;
c) hydrolyse enzymatique du substrat prétraité et éventuellement lavé utilisant des enzymes cellulolytiques et/ou hémicellulolytiques produisant un hydrolysat et un résidu insoluble dans l'eau;
d) fermentation par microorganisme de l'hydrolysat issu de l'étape c) et obtention d'un moût de fermentation contenant au moins un alcool et/ou solvant ;
e) séparation/purification de l'alcool et/ou solvant et
f) séparation d'un gâteau contenant le résidu insoluble,
dans lequel au moins une partie du gâteau obtenu à l'étape f) est envoyée dans
au moins un réacteur de régénération de la cellulose, avant d'être recyclée en aval de l'étape a) de prétraitement chimique alcalin, la fraction du gâteau extrait à l'étape f) qui est envoyée dans le réacteur de régénération est mélangée à une solution alcaline, puis chauffée à une température comprise entre 50 et 150 °C.

Ainsi, grâce au procédé selon la présente invention, il est possible d'améliorer la valorisation du substrat lignocellulosique. En effet, le procédé permet d'utiliser plus de 80% poids, et de préférence plus de 90% poids de la cellulose contenue dans le végétal pour sa future conversion alcoolique et/ou en mélange ABE.
Grâce au traitement thermique réalisé en milieu alcalin, dans des conditions douces, dans un réacteur spécifique dit de régénération de la cellulose, la cellulose non hydrolysée, encore appelée cellulose récalcitrante, retrouve partiellement sa susceptibilité à l'hydrolyse enzymatique. Sous le terme de cellulose récalcitrante, au sens de la présente invention, on entend la cellulose non hydrolysée au cours de l'étape c) d'hydrolyse enzymatique et qui présente, sans traitement spécifique, une susceptibilité médiocre à l'hydrolyse enzymatique.

Selon le procédé de la présente invention, une fraction du gâteau extrait à l'étape f) est envoyée dans au moins un réacteur de régénération de la cellulose avant d'être recyclée en aval de l'étape a) de prétraitement alcalin : la cellulose récalcitrante non hydrolysée subit ainsi un traitement thermique, en milieu alcalin, dans des conditions plus douces que celles mises en oeuvre lors de l'étape de prétraitement. Ce traitement permet notamment le gonflement des fibres de la pâte et régénère la susceptibilité du substrat à l'hydrolyse enzymatique, sans entraîner d'accumulation de lignine. La fraction du gâteau extrait à l'étape f) qui est envoyée dans le réacteur de régénération est mélangée à une solution alcaline, puis chauffée à une température comprise entre 50 et 150°C, de préférence pendant une durée variant entre 10 min et 4 heures.

La solution alcaline est préférentiellement du sulfate de sodium. La température de cuisson dans le réacteur de régénération est alors comprise entre 70 et 150°C, de préférence pour un temps de séjour dans le réacteur compris entre 0,5 et 4 heures.

La solution alcaline peut également être de l'ammoniac gazeux. Dans ce cas, la température est comprise entre 50 et 100°C, de préférence pour un temps de séjour compris entre 10 et 60 minutes.

La solution alcaline peut encore être une solution ammoniacale percolée, chauffée à une température comprise entre 80 et 140°C dans le réacteur de régénération.

Le procédé selon la présente invention permet de limiter la quantité d'enzymes à utiliser pour parvenir à une hydrolyse globale supérieure à 90% de la cellulose du substrat initial prétraité. In fine, l'enzyme ne rencontre qu'un substrat "très susceptible à l'hydrolyse enzymatique" et ne rencontre plus de cellulose récalcitrante du fait du recyclage de celle-ci.

L'invention va être décrite en se référant à la Figure 1.

Le substrat utilisé est choisi parmi les biomasses les plus variées, mais plus particulièrement depuis les espèces arborescentes résineuses (softwood tels que les épicéas ou les pins) ou feuillues (hardwood tels que les eucalyptus) ou encore les déchets lignocellulosiques agricoles (paille de blé, riz, etc.).

Le prétraitement réalisé à l'étape a) est un prétraitement de type alcalin. Cette première étape est une étape de cuisson du substrat cellulosique ou lignocellulosique en présence d'un réactif chimique alcalin. Ce réactif se présente sous forme liquide ou gazeuse, en fonction du prétraitement mis en oeuvre.

Selon un mode de réalisation, le prétraitement chimique alcalin réalisé à l'étape a) est préférentiellement un prétraitement au sulfate de sodium (ou procédé Kraft) classiquement utilisé dans les procédés de production de produits papetiers, dit Kraft ou "pâte au sulfate", à l'issue duquel on obtient des pâtes papetières.

Le procédé au sulfate de sodium ou procédé Kraft est basé sur l'utilisation de soude et de sulfate de sodium. Le traitement chimique des copeaux de bois se fait à 150-180°C pendant une durée de 1 à 7 heures en fonction du substrat utilisé. Les pâtes papetières kraft sont produites à partir des biomasses les plus variées, mais plus particulièrement depuis les espèces arborescentes résineuses (softwood tels que les épicéas ou les pins) ou feuillues (hardwood tels que les eucalyptus) ou encore les déchets lignocellulosiques agricoles (paille de blé, riz, etc.). Elles sont partiellement délignifiées au moyen de cuissons à haute température et en présence de soude. Cette délignification est contrôlée par les paramètres opératoires des réacteurs. La cuisson est réalisée dans un réacteur vertical, où les copeaux descendent par gravité et rencontrent les diverses liqueurs de cuisson. Le sulfure de sodium est préparé directement à partir de sulfate de sodium par combustion. Lors de la cuisson, le sulfure de sodium est hydrolysé en soude, en NaHS et en H₂S. Les différents composés soufrés présents réagissent avec la lignine pour donner des thiolignines plus facilement solubles. La liqueur appliquée aux copeaux est appelée liqueur blanche. La liqueur extraite du réacteur ou lessiveur contenant les composés éliminés de la paroi est appelée liqueur noire.

La biomasse est introduite par une conduite 1 dans le réacteur de cuisson ou lessiveur 2. La liqueur blanche y est également introduite via la conduite 3. La biomasse est partiellement délignifiée au moyen de cuissons à haute température et en présence de soude. La lignine solubilisée est retirée avec la solution alcaline et est évacuée par la conduite 4 avec la liqueur noire.

Cette étape de délignification peut avoir lieu dans plusieurs lessiveurs successifs non représentés sur la figure et est contrôlée par les paramètres opératoires fixés dans ces dispositifs.

La pâte obtenue en sortie des lessiveurs circulant dans la conduite 5 est enrichie en cellulose : elle contient entre 60 et 90 % en poids de cellulose par rapport à la matière solide totale et entre 5 et 20% d'hémicellulose.

Selon d'autres modes de réalisation, le prétraitement chimique alcalin réalisé à l'étape a) peut également être un prétraitement par explosion des fibres à l'ammoniac, encore appelé prétraitement AFEX (Ammonia Fiber Explosion) ou un prétraitement par percolation utilisant de l'ammoniaque avec recycle, encore appelé prétraitement ARP (Ammonia Recycle Percolation).

Le procédé ARP (Ammonia Recycle Percolation) est un procédé de prétraitement utilisant de l'ammoniaque avec recyclage. Ce type de procédé est notamment décrit par Kim et al., 2003, Biores. Technol. 90 (2003) p 39-47. La température élevée de la percolation conduit à une solubilisation partielle à la fois de la lignine et des hémicelluloses, cette solution est ensuite chauffée pour recycler l'ammoniaque et récupérer d'une part la lignine extraite, par exemple pour une valorisation énergétique, et d'autre part les sucres solubles issus des hémicelluloses.

Selon ce mode de réalisation, dans le cas d'un prétraitement ARP, la biomasse est introduite par la conduite 1 dans le réacteur de cuisson 2. Une solution ammoniacale est percolée sur la biomasse sous pression (15 à 30 bars) et à température élevée, 130°C à 190°C. La biomasse est partiellement délignifiée, une partie des hémicelluloses est aussi solubilisée. Les sucres et la lignine solubilisés sont retirés avec la solution alcaline usée et sont évacués par la conduite 4.

Le procédé AFEX (Ammonia Fiber Explosion) consiste à introduire le substrat lignocellulosique dans un cuiseur à haute pression en présence d'ammoniac, puis de provoquer une détente explosive en sortie du réacteur et de recycler l'ammoniac alors sous forme gazeuse. Ce type de procédé est notamment décrit par Teymouri et al., 2005, Biores. Technol. 96 (2005) p.2014-2018. Ce procédé conduit principalement à une déstructuration de la matrice de la biomasse mais il n'y a pas de séparation phasique des composés lignine, hémicellulose et cellulose en sortie de traitement.

Selon cet autre mode de réalisation, dans lequel le prétraitement est de type AFEX, la biomasse est introduite par la conduite 1 dans un réacteur de cuisson 2. Une solution ammoniacale est introduite par la conduite 3 sous pression, de 15 à 30 bars, à température modérée (70°C à 110°C). Le mélange est maintenu dans ces conditions durant un temps déterminé en fonction du substrat, puis détendu en sortie du cuiseur. La solution ammoniacale est récupérée via la conduite 4 sous forme gazeuse pour être recyclée. Le substrat prétraité extrait par la conduite 5 du cuiseur a essentiellement la même composition que le substrat en entrée.

Le substrat prétraité selon un procédé AFEX ou ARP circulant dans la conduite 9 comprend entre 50 et 95% poids de matières insolubles dans l'eau, et plus particulièrement entre 60 et 85% poids de matières insolubles dans l'eau.

D'autres traitements alcalins sont aussi à l'étude, notamment à base de soude ou de chaux, une revue non exhaustive est donnée par Ogier et al., 1999, Oil & Gas Science and Technology- Revue de l'IFP, Vol. 54 (1999), No. 1, pp. 67-94.

Ces différents prétraitements alcalins peuvent être combinés à une action mécanique, créée par exemple dans une extrudeuse de type bi-vis ou une défribreuse.

A l'issue de l'étape a) de prétraitement selon le procédé de la présente invention, on obtient le substrat prétraité sous forme d'une pâte 5 (encore appelée "pulpe") enrichie en cellulose.

Lors de l'étape b) de lavage du substrat prétraité, cette pâte circulant dans la conduite 5 est éventuellement lavée dans le réacteur 6. Un ou plusieurs liquides de lavage 7 sont introduits dans ledit réacteur 6. Cette étape de lavage peut également être répétée plusieurs fois, éventuellement dans plusieurs réacteurs de lavage successifs. Elle peut également se limiter à une étape de dilution.

Une délignification plus poussée peut être conduite lors de l'étape de lavage réalisée dans le réacteur 6. Un outil de séparation comme une presse ou un décanteur centrifuge peut être installé pour éliminer l'alcalinité.

L'étape b) de lavage est nécessaire si le prétraitement alcalin est un prétraitement de type Kraft ou ARP.

Le ou les liquides de lavage usés 8 sont retirés en sortie du réacteur 6.

Dans le cas d'un prétraitement de type Kraft, la pâte ou pulpe lavée 9 qui est extraite du réacteur de lavage 6 contient entre 1% et 40% de matière solide, de préférence entre 7% et 40%, et plus préférentiellement entre 10% et 25%.

Dans le cas d'un traitement AFEX, l'étape de lavage b) après ce prétraitement peut se limiter à une étape de dilution avec un liquide de dilution introduit par la conduite 7, auquel cas le flux de liquide de lavage évacué par la conduite 8 est nul.

Une neutralisation de la pâte peut être conduite préalablement à l'étape d'hydrolyse enzymatique par addition d'acides. Il est en effet nécessaire que l'hydrolyse enzymatique soit réalisée à un pH compris entre 4 et 5,5.

La pâte prétraitée et éventuellement lavée et neutralisée est ensuite envoyée dans le procédé de conversion en alcools et / ou solvants représenté schématiquement par le rectangle 10, où sont réalisées les étapes c) à e), correspondant aux étapes de conversion proprement dites. Ces étapes de conversion peuvent être au nombre de deux à huit. De préférence, on en compte entre trois et cinq.
Ces étapes de conversion comprennent au moins les étapes c) et d) correspondant respectivement à une hydrolyse enzymatique et une fermentation de la pulpe. Ces étapes peuvent éventuellement être couplées dans un même réacteur. On parle alors de procédé SSF ("Simultaneous Saccharification and Fermentation").

L'étape c) d'hydrolyse enzymatique est réalisée au moyen des enzymes de type cellulases et/ou hémicellulases produites par un microorganisme. De façon préférée, le microorganisme utilisé est un champignon appartenant aux genres *Trichoderma, Aspergillus, Penicillium* ou *Schizophyllum,* ou une bactérie anaérobie appartenant au genre *Clostridium.* De façon très préférée, le microorganisme utilisé est *Trichoderma reesei.* Il est produit dans une ligne de production indépendante qui peut être réalisée sur site ou hors site.

Grâce au prétraitement alcalin, la susceptibilité à l'hydrolyse enzymatique est excellente et les polymères de cellulose et d'hémicellulose sont convertis en sucres dits "très fermentescibles" (glucose, mannose), "médiocrement fermentescible" (galactose), "difficilement fermentescibles", (xylose et arabinose). Les conditions de l'hydrolyse enzymatique, principalement le taux de matière sèche du mélange à hydrolyser et la quantité d'enzymes utilisée, sont choisies de façon à ce que l'étape c) est réalisée de sorte qu' une conversion entre 20% et 90% de la cellulose de la pulpe circulant dans la conduite 9 en glucose, et plus particulièrement entre 30% et 80% soit obtenue.

La fermentation alcoolique réalisée à l'étape d) est assurée par des levures ou autres microorganismes.

Lors de l'étape e), les alcools et/ou solvants produits à l'étape d) dont purifiés et séparés.

L'étape f) de séparation du gâteau peut être réalisée en aval des étapes c), d) et/ou e) et peut éventuellement être couplée à un lavage du gâteau.

Dans tous les cas, en sortie des étapes c) à e) réalisées dans le réacteur 10, on obtient un flux de produits 11, éventuellement séparés par tout moyen connu de l'homme de l'art, un résidu liquide 12 (appelé vinasses) contenant des sucres non fermentés et un gâteau solide 13 contenant de la matière solide issue du substrat initial (résidu solide) et une fraction liquide. Le résidu solide est en partie composé de cellulose qui n'a pas été hydrolysée, et qui représente entre 10% et 100% du solide, et de préférence entre 30% et 70%.

Le flux 13 correspondant au gâteau est divisé en 2 fractions 13-1 et 13-2.

La fraction 13-1 est envoyée vers le réacteur 14 de régénération de la cellulose. Cette fraction représente entre 20 et 100% du gâteau 13, et de préférence entre 75 et 100% et encore plus préférentiellement entre 85 et 100%.

Une solution alcaline est introduite dans le réacteur 14 par la conduite 15, pour être mélangée avec la fraction 13-1.

Le flux sortant de ce réacteur est recyclé par la conduite 17 en aval de l'étape a) de prétraitement chimique alcalin.

Ainsi, grâce au traitement dans le réacteur de régénération permettant notamment le gonflement des fibres, la cellulose récalcitrante contenue dans le flux 13-1 retrouve partiellement sa susceptibilité à l'hydrolyse enzymatique

La fraction 13-1 non recyclée est directement évacuée en dehors du procédé. Elle représente entre 0 et 80% du gâteau 13, et de préférence moins de 25%, et encore mieux moins de 15%.

### EXEMPLES

Dans tous les exemples ci-dessous, on note ms: matière sèche.
FPu = *Filter Paper unit,* ou unité papier filtre, qui est une mesure de l'activité enzymatique. La correspondance FPu - poids est une caractéristique du cocktail enzymatique.

### Exemple 1 : Bilan matière -sans recyclage (non conforme à l'invention)

On considère un procédé de production d'éthanol à partir de pulpe papetière issue d'un procédé alcalin Kraft. Le procédé traite 80 tonnes / heure de végétal natif. Le végétal est de l'épicéa (softwood), contenant 55 % poids de matière sèche constituée de :

| | |
|---|---|
| cellulose | 42% |
| lignine | 30% |
| hemicellulose | 15% |
| autres (cendres, extractibles...) | 13% |

Les hémicelluloses sont constitués pour moitié de mannanes.
La cuisson Kraft s'effectue à 175°C pendant 5 heures. Ce prétraitement et les procédés de lavage réalisés aux étapes a) et b) respectivement sont conduits de telle sorte que la pulpe papetière contient 15% de matière sèche, et a conservé:

| | |
|---|---|
| cellulose | 97% |
| lignine | 10% |
| hemicellulose | 52% |
| autres | 8% |

Le procédé de conversion en éthanol consiste en une hydrolyse enzymatique de la pulpe papetière (étape c)), suivie d'une fermentation alcoolique en éthanol (étape d)), d'une séparation des solides en suspension pour former un gâteau, de la distillation puis déshydratation de l'éthanol à 99,7% poids (étape e)).

L'hydrolyse enzymatique est menée dans des conditions telles qu'on observe l'hydrolyse de 75% de la cellulose et de 55% des hémicelluloses. On consomme 20 FPu / g de cellulose entrant dans le réacteur d'hydrolyse.

La fermentation permet de transformer 90% du glucose et du mannose formés précédemment en éthanol. Les autres sucres issus des hémicelluloses (xylose, arabinose,...) ne sont pas fermentés par la souche de *Saccharomyces cerevisae* utilisée.
Avant l'étape de distillation, le résidu solide est séparé et lavé pour limiter la perte en éthanol avec le gâteau.

Les conditions du procédé sont telles que les flux en sortie sont:
- éthanol à 99,7% poids: 6,96 tonnes / heure
- vinasses: 143,64 tonnes / heure
- gâteau solide: 22,96 tonnes / heure à 36% de matière solide. La partie solide est pour 54,1% de la cellulose non hydrolysée.

Le rendement en éthanol de ce procédé est donc de 15,8% poids sur le végétal natif (base matière sèche). La consommation spécifique d'enzymes est de 51 540 FPu / kg d'éthanol produit.
La cellulose dite "récalcitrante" qui est présente dans le gâteau, a un rendement d'hydrolyse (dans les conditions du procédé ci-dessus), et avec la même charge enzymatique qui ne sera que de 30%.

### Exemple 2: Bilan matière - avec recyclage du résidu solide au niveau de l'hydrolyse enzymatique (non conforme à l'invention)

Il est possible de recycler le gâteau au niveau de l'étape d'hydrolyse enzymatique afin de limiter les pertes en cellulose. Néanmoins, en l'absence de traitement, la cellulose dite "récalcitrante" a une sensibilité à l'hydrolyse enzymatique très réduite par rapport à la cellulose de la pâte papetière. Son rendement d'hydrolyse (dans les conditions du procédé ci-dessus), et avec la même charge enzymatique, ne sera que de 30%. De plus, le recyclage entraîne l'accumulation des insolubles (lignine) dans le procédé et la fermentation, dans les conditions du procédé ci-dessus, doit être effectuée avec un maximum de 8% de matière solide dans le milieu réactionnel. De ce fait, il est nécessaire de limiter la quantité de gâteau recyclé, et en pratique, seul 68% du gâteau peut être recyclé. On obtient alors les flux suivants en sortie du procédé:
- éthanol à 99,7% poids: 7,92 tonnes / heure, soit 14% de plus que l'exemple 1.
- vinasses: 198,06 tonnes / heure
- gâteau solide: 17,14 tonnes / heure à 36% de matière solide (partie non recyclée). La partie solide est pour 50,3% de la cellulose non hydrolysée.

Le rendement en éthanol de ce procédé est donc de 18,0% poids sur le végétal natif (base matière sèche), soit une amélioration de 2,2 points par rapport au cas de base. Néanmoins, cette amélioration du bilan massique se fait au détriment de la consommation spécifique d'enzymes qui est alors de 61 740 FPu / kg d'éthanol produit (+20%), et nécessite un volume réactionnel plus important: +56% pour l'hydrolyse enzymatique, soit un augmentation du volume spécifique (rapporté à la production) de 36%.

Ainsi, l'amélioration du bilan massique permet de diminuer la contribution du coût de la matière première dans le coût final de production de l'éthanol, mais les postes de dépenses "enzymes" et "investissements" sont augmentés de façon importante.

### Exemple 3: Bilan matière - avec recyclage au sein d'un réacteur de régénération (selon l'invention)

Sur la base du procédé décrit dans l'exemple 1, on introduit un recyclage de 90% du gâteau créé dans un réacteur de régénération, où le gâteau subit une cuisson "douce" à 110°C pendant 1 heure en présence de sulfate de sodium, avant d'être mélangé au végétal natif prétraité en aval du réacteur 2 de prétraitement.
On recycle ainsi 27,18 tonnes / heure de gâteau humide contenant 52,2% de cellulose au niveau du réacteur de régénération. Le coût de ce type de cuisson est inférieur à celui du traitement sévère réalisé dans le réacteur 2 pour le prétraitement du végétal natif. Par ailleurs, la cellulose recyclée n'est plus beaucoup protégée par la lignine, puisque la gaine ligneuse protégeant les fibres a été en grande partie retirée lors du prétraitement, et donc une cuisson plus douce et plus courte est suffisante pour rendre aux fibres de cellulose leur pleine susceptibilité à l'hydrolyse enzymatique, et permet de limiter fortement les pertes de matière. Lors des étapes de cuisson dédiée et de lavage, on conserve:

| | |
|---|---|
| cellulose | 95% |
| lignine | 40% |
| hemicellulose | 60% |
| autres (cendres, extractibles...) | 10% |

Les conditions d'hydrolyse et de fermentation sont conservées. Du fait du gonflement important des fibres de cellulose recyclées en milieu alcalin et en l'absence de lignine entourant ces fibres à leur entrée dans le réacteur de régénération, la cellulose retrouve toute sa susceptibilité à l'hydrolyse enzymatique et a donc un rendement d'hydrolyse égal à celui de la cellulose issu du végétal natif (75%). Les hémicelluloses retrouvent aussi un rendement de 55%. Les conditions de fermentation alcoolique et de séparation sont maintenues. On obtient ainsi grâce au procédé selon l'invention des flux en sortie du procédé:
- éthanol à 99,7% poids: 8,79 tonnes / heure, soit 26% de plus que l'exemple 1.
- vinasses: 177,10 tonnes / heure
- gâteau solide: 3,02 tonnes / heure à 36% de matière solide (partie non recyclée). La partie solide est pour 52,2% de la cellulose non hydrolysée.

Le rendement en éthanol de ce procédé est donc de 20,0% poids sur le végétal natif (base matière sèche), soit 4,2 points de plus que l'exemple 1 et 2 points de plus que l'exemple 2. Par ailleurs, la consommation spécifique d'enzymes n'a que très légèrement augmentée et est de 51 750 FPu / kg d'éthanol produit, soit seulement 0,4% d'augmentation. Le volume réactionnel mis en jeu est de 29% supérieur à l'exemple 1, et donc le volume spécifique n'a que légèrement augmenté par rapport à l'exemple 1 (+1,7%).
La mise en oeuvre du procédé selon l'invention a permis d'améliorer fortement le bilan massique et donc de diminuer la contribution du coût de la matière première dans le coût final de production de l'éthanol. Le recycle suivant l'invention permet de contrôler le niveau de lignine dans le procédé, et donc permet de recycler une plus grande quantité que l'exemple 2, tout en gardant un niveau correct de solides en fermentation, ce qui conduit a un rendement matière encore meilleur. De plus, l'invention permet de maintenir la contribution des postes de dépenses "enzymes" et "investissements" au niveau du cas sans recycle (moins de 2% d'augmentation). Les conditions "douces" de cuisson permettent de conserver une très grande partie de la cellulose tout en lui rendant sa susceptibilité à l'hydrolyse par les enzymes, et le coût associé à cette cuisson est plus faible que celui du prétraitement du végétal natif.

### Exemple 4 : Bilan matière -sans recyclage (non conforme à l'invention)

On considère un procédé de production d'un mélance Acétone-Butnaol-Ethanol (ABE) à partir de pulpe papetière issue d'un procédé alcalin Kraft. Le procédé traite 150 tonnes / heure de végétal natif. Le végétal est de l'eucalyptus (hardwood), contenant 50 % poids de matière sèche constituée de :

| | |
|---|---|
| cellulose | 45% |
| lignine | 22% |
| hemicellulose | 17% |
| autres (cendres, extractibles....) | 16% |

Les hémicelluloses sont constitués du sucres en C5 (xylans et arabinanes).
La cuisson Kraft s'effectue à 165°C pendant 2,5 heures. Ce prétraitement et les procédés de lavage réalisés aux étapes a) et b) respectivement sont conduits de telle sorte que la pulpe papetière contient 10% de matière sèche, et a conservé:

| | |
|---|---|
| cellulose | 98,5% |
| lignine | 9% |
| hemicellulose | 65% |
| autres | 20% |

Le procédé de conversion en éthanol consiste en une hydrolyse enzymatique de la pulpe papetière (étape c), d'une séparation des solides en suspension pour former un gâteau avec un lavage pour maximiser la récupération les sucres, puis d'une fermentation en ABE de la phase liquide contenant les sucres (étape d)), de la distillation de l'ABE (étape e). Il faut noter que la fermentation en ABE utilise à la fois les sucres à 6 atomes et à 5 atomes de carbone (glucose et xylose).

L'hydrolyse enzymatique est menée dans des conditions telles qu'on observe l'hydrolyse de 85% de la cellulose et de 65% des hémicelluloses. On consomme 25 FPu / g de cellulose entrant dans le réacteur d'hydrolyse.

Avant l'étape de fermentation, le résidu solide est séparé et lavé pour limiter la perte en sucres avec le gâteau.

La fermentation permet de transformer le glucose et le xylose formés précédemment en mélange ABE, produisant 0,3 g d'ABE par g de sucres présent.

Les conditions du procédé sont telles que les flux en sortie sont:
- ABE (pur): 11,16 tonnes / heure
- vinasses: 416,95 tonnes / heure
- gâteau solide: 33,74 tonnes / heure à 33,3% de matière solide. La partie solide est pour 44,3% de la cellulose non hydrolysée.

Le rendement en ABE de ce procédé est donc de 14,9% poids sur le végétal natif (base ms). La consommation spécifique d'enzymes est de 74 470 FPu / kg d'ABE produit.
La cellulose dite "récalcitrante" qui est présente dans le gâteau, a un rendement d'hydrolyse (dans les conditions du procédé ci-dessus), et avec la même charge enzymatique qui ne sera que de 25%.

### Exemple 5: Bilan matière - avec recyclage du résidu solide au niveau de l'hydrolyse enzymatique (non conforme à l'invention)

Il est possible de recycler le gâteau au niveau de l'étape d'hydrolyse enzymatique afin de limiter les pertes en cellulose. Néanmoins, en l'absence de traitement, la cellulose dite "récalcitrante" a une sensibilité à l'hydrolyse enzymatique très réduite par rapport à la cellulose de la pâte papetière. Son rendement d'hydrolyse (dans les conditions du procédé ci-dessus), et avec la même charge enzymatique, ne sera que de 25%. De plus, le recyclage entraîne l'accumulation des insolubles (lignine) dans le procédé, ce qui conduit à des volumes de réacteurs d'hydrolyse plus importants. On recycle 90% du gâteau ainsi formé. On obtient alors les flux suivants en sortie du procédé:
- ABE (pur): 12,84 tonnes / heure, soit 15% de plus que l'exemple 4.
- vinasses: 787,41 tonnes / heure
- gâteau solide: 17,10 tonnes / heure à 33,3% de matière solide (partie non recyclée). La partie solide est pour 26,9% de la cellulose non hydrolysée.

Le rendement en ABE de ce procédé est donc de 17,1% poids sur le végétal natif (base matière sèche), soit une amélioration de 2,2 points par rapport au cas de base. Néanmoins, cette amélioration du bilan massique se fait au détriment de la consommation spécifique d'enzymes qui est alors de 91 590 FPu / kg d'ABE produit (+23%), et nécessite un volume réactionnel qui a plus que doublé: +113% pour l'hydrolyse enzymatique, soit un augmentation du volume spécifique (rapporté à la production) de +85%.
Ainsi, l'amélioration du bilan massique permet de diminuer la contribution du coût de la matière première dans le coût final de production de l'ABE, mais les postes de dépenses "enzymes" et surtout "investissements" sont augmentés de façon importante.

### Exemple 6: Bilan matière - avec recyclage du résidu solide dans un réacteur de régénération (selon l'invention).

Sur la base du procédé décrit dans l'Exemple 4, on introduit un recyclage de 90% du gâteau créé dans un réacteur de régénération, où le gâteau subit une cuisson "douce" à 105°C pendant 45 minutes, en présence de sulfate de sodium, avant d'être mélangé au végétal natif prétraité en sortie du réacteur 2 de prétraitement.
On recycle ainsi 38,52 tonnes / heure de gâteau humide contenant 40,2% de cellulose au niveau du réacteur de régénération. Le coût de ce type de cuisson est inférieur à celui du traitement sévère réalisé dans le réacteur de prétraitement 2 pour le prétraitement du végétal natif. Par ailleurs, la cellulose recyclée n'est plus beaucoup protégée par la lignine, puisque la gaine ligneuse protégeant les fibres a été en grande partie retirée lors du prétraitement, et donc une cuisson plus douce et plus courte est suffisante pour rendre aux fibres de cellulose leur pleine susceptibilité à l'hydrolyse enzymatique, et permet de limiter fortement les pertes de matière. Lors des étapes de cuisson dédiée et de lavage, on conserve:

| | |
|---|---|
| cellulose | 97% |
| lignine | 50% |
| hemicellulose | 65% |
| autres | 18% |

Les conditions d'hydrolyse et de fermentation sont conservées. L'hydrolyse du végétal natif a le même rendement. Du fait du gonflement important des fibres de cellulose recyclées en milieu alcalin et en l'absence de lignine entourant ces fibres à leur entrée dans le lessiveur pour le prétraitement alcalin chimique, la cellulose retrouve toute sa susceptibilité à l'hydrolyse enzymatique et a donc un rendement d'hydrolyse égal à celui de la cellulose issu du végétal natif (85%). Les hémicelluloses retrouvent aussi un rendement de 65%. Les conditions de séparation et de fermentation ABE sont maintenues. On obtient ainsi grâce au procédé selon l'invention des flux en sortie du procédé:
- ABE (pur): 12,98 tonnes / heure, soit 16,3% de plus que l'exemple 4.
- vinasses: 489,05 tonnes / heure
- gâteau solide: 4,28 tonnes / heure (partie non recyclé), contenant 33,3% de solides. La partie solide est pour 40,2% de la cellulose.

Le rendement en ABE de ce procédé est donc de 17,3% poids sur le végétal natif (base matière sèche), soit 2,4 points de plus que l'exemple 4 et 0,2 point de plus que l'exemple 5. Par ailleurs, la consommation spécifique d'enzymes a très légèrement diminuée et est de 73 690 FPu / kg d'ABE produit, soit 1 % de réduction. Le volume réactionnel mis en jeu est de 19,1% supérieur à l'exemple 4, et donc le volume spécifique est légèrement supérieur que pour l'exemple 4 (+2,4%).
La mise en oeuvre du procédé selon l'invention a permis d'améliorer fortement le bilan massique et donc de diminuer la contribution du coût de la matière première dans le coût final de production de l'ABE. Le recycle suivant l'invention permet de contrôler le niveau de lignine dans le procédé, et donc permet de limiter le volume nécessaire à l'hydrolyse par rapport à l'exemple 5. De plus, l'invention permet de maintenir la contribution des postes de dépenses "enzymes" et "investissements" au niveau du cas sans recycle (moins de 3% d'écart). Les conditions "douces" de cuisson permettent de conserver une très grande partie de la cellulose tout en lui rendant sa.susceptibilité à l'hydrolvse par les enzymes, et le coût associé à cette cuisson est plus faible que celui du pretraitement du végétal natif.

## Revendications

1. Procédé de production d'alcools et/ou de solvants à partir de biomasse cellulosique ou lignocellulosique comprenant au moins les étapes suivantes :
a) prétraitement chimique alcalin d'un substrat cellulosique ou lignocellulosique ;
b) éventuellement lavage du substrat prétraité ;
c) hydrolyse enzymatique du substrat prétraité et lavé utilisant des enzymes cellulolytiques et/ou hémicellulolytiques produisant un hydrolysat et un résidu insoluble dans l'eau;
d) fermentation par microorganisme de l'hydrolysat issu de l'étape c) et obtention d'un moût de fermentation contenant au moins un alcool et/ou solvant ;
e) séparation/purification de l'alcool et/ou solvant et
f) séparation d'un gâteau contenant le résidu insoluble,
dans lequel au moins une partie (13-1) du gâteau (13) obtenu à l'étape f) est envoyée dans au moins un réacteur de régénération de la cellulose, avant d'être recyclée en aval de l'étape a) de prétraitement chimique alcalin, la fraction du gâteau extrait à l'étape f) qui est envoyée dans le réacteur de régénération est mélangée à une solution alcaline, puis chauffée à une température comprise entre 50 et 150°C.

2. Procédé de production selon la revendication 1 dans lequel au moins une fraction (13-1) représentant entre 20 et 100% du gâteau (13) est envoyée dans ledit réacteur de régénération.

3. Procédé selon la revendication 2 dans lequel la fraction (13-1) représente entre 75 et 100% du gâteau (13).

4. Procédé selon l'une des revendications 1 à 3 dans lequel au moins une fraction (13-2) représentant entre 0 et 80% du gâteau est directement évacuée sans recyclage.

5. Procédé selon la revendication 4 dans lequel la fraction (13-2) représente moins de 25% du flux du résidu solide (13).

6. Procédé selon l'une des revendications 1 à 5 dans lequel la fraction (13-1) est mélangée à une solution alcaline dans le réacteur de régénération, puis chauffée à une température comprise entre 70 et 150°C, de préférence pendant une durée variant entre 10 min et 4 heures.

7. Procédé selon la revendication 6 dans lequel ladite solution alcaline est du sulfate de sodium, la température dans ledit réacteur étant comprise entre 70 et 150 °C, et de préférence le temps de séjour compris entre 0,5 et 4 heures.

8. Procédé selon la revendication 6 dans lequel ladite solution alcaline est de l'ammoniac gazeux, la température étant comprise entre 50 et 100°C, et de préférence le temps de séjour entre 10 et 60 minutes.

9. Procédé selon la revendication 6 dans lequel ladite solution alcaline est une solution ammoniacale percolée, chauffée à une température comprise entre 80 et 140°C.

10. Procédé selon l'une des revendications 1 à 9 dans lequel le prétraitement réalisé à l'étape a) est un prétraitement au sulfate de sodium, encore appelé procédé Kraft, un prétraitement par explosion des fibres à l'ammoniac, encore appelé prétraitement AFEX ou un prétraitement par percolation utilisant de l'ammoniaque avec recycle, encore appelé prétraitement ARP.

11. Procédé selon l'une des revendications 1 à 10 dans lequel l'étape b) de lavage est nécessaire si le prétraitement alcalin est un prétraitement de type Kraft ou ARP.

12. Procédé selon l'une des revendications 1 à 11 dans lequel l'étape c) d'hydrolyse enzymatique est réalisée au moyen des enzymes de type cellulases et/ou hémicellulases produites par un microorganisme qui est un champignon appartenant aux genres *Trichoderma, Aspergillus, Penicillium* ou *Schizophyllum,* ou une bactérie anaérobie appartenant au genre *Clostridium.*

13. Procédé selon l'une des revendications 1 à 12 dans lequel l'étape c) est réalisée de façon à ce que entre 20 et 90%, et préférentiellement entre 30 et 80% de la cellulose contenue dans la pâte prétraitée et lavée soit convertie en glucose.

14. Procédé selon l'une des revendications 1 à 13 dans lequel l'alcool obtenu à l'issue de l'étape e) est de l'éthanol.

15. Procédé selon l'une des revendications 1 à 13 dans lequel le solvant obtenu à l'issue de l'étape e) est un mélange acétone-butanol-éthanol.

16. Procédé selon l'une des revendications 2 à 11 dans lequel l'étape f) de séparation du gâteau est réalisée en aval des étapes c), d) et/ou e) et éventuellement est couplée à un lavage du gâteau.

## Patentansprüche

1. Verfahren zur Herstellung von Alkoholen und/oder Lösungsmitteln aus zellulosehaltiger oder lignozellulosehaltiger Biomasse, das mindestens die folgenden Schritte umfasst:
a) alkalisch-chemisches Vorbehandeln eines zellulosehaltigen oder lignozellulosehaltigen Substrats;
b) gegebenenfalls Waschen des vorbehandelten Substrats;
c) enzymatisches Hydrolysieren des vorbehandelten und gewaschenen Substrats unter Verwendung der zellulosespaltenden und/oder hemizellulosespaltenden Enzyme, die ein Hydrolysat und einen in Wasser unlöslichen Rückstand erzeugen;
d) Fermentieren mit Mikroorganismus des aus Schritt c) stammenden Hydrolysats und Erhalten einer Fermentationsmaische, die mindestens einen Alkohol und/oder ein Lösungsmittel enthält;
e) Abtrennen/Reinigen des Alkohols und/oder des Lösungsmittels und
f) Abtrennen eines Kuchens, der den unlöslichen Rückstand enthält,
wobei mindestens ein Teil (13-1) des in Schritt f) erhaltenen Kuchens (13) in mindestens einen Reaktor zur Regeneration der Zellulose geschickt wird, bevor er stromabwärts von Schritt a) zur alkalischchemischen Vorbehandlung recycelt wird, wobei die Fraktion des in Schritt f) extrahierten Kuchens, der in den Reaktor zur Regeneration geschickt wird, mit einer alkalischen Lösung gemischt, dann auf eine Temperatur im Bereich zwischen 50 und 150 °C erhitze wird.

2. Herstellungsverfahren nach Anspruch 1, wobei mindestens eine Fraktion (13-1), die zwischen 20 und 100 % des Kuchens (13) ausmacht, in den Regenerationsreaktor geschickt wird.

3. Verfahren nach Anspruch 2, wobei die Fraktion (13-1) zwischen 75 und 100 % des Kuchens (13) ausmacht.

4. Verfahren nach einem der Ansprüche 1 bis 3, wobei mindestens eine Fraktion (13-2), die zwischen 0 und 80 % des Kuchens ausmacht, ohne Recycling direkt abgeleitet wird.

5. Verfahren nach Anspruch 4, wobei die Fraktion (13-2) weniger als 25 % des festen Rückstandsstroms (13) ausmacht.

6. Verfahren nach einem der Ansprüche 1 bis 5, wobei die Fraktion (13-1) in dem Regenerationsreaktor mit einer alkalischen Lösung gemischt, dann auf eine Temperatur im Bereich zwischen 70 und 150 °C, bevorzugt für eine Dauer, die zwischen 10 Min. und 4 Stunden variiert, erwärmt wird.

7. Verfahren nach Anspruch 6, wobei die alkalische Lösung Natriumsulfat ist, die Temperatur in dem Reaktor im Bereich zwischen 70 und 150 °C liegt und die Verweildauer bevorzugt im Bereich zwischen 0,5 und 4 Stunden liegt.

8. Verfahren nach Anspruch 6, wobei die alkalische Lösung gasförmiger Ammoniak ist, die Temperatur im Bereich zwischen 50 und 100 °C liegt und die Verweildauer bevorzugt im Bereich zwischen 10 und 60 Minuten liegt.

9. Verfahren nach Anspruch 6, wobei die alkalische Lösung eine auf eine Temperatur im Bereich zwischen 80 und 140 °C erwärmte, perkolierte Ammoniaklösung ist.

10. Verfahren nach einem der Ansprüche 1 bis 9, wobei es sich bei der in Schritt a) durchgeführten Vorbehandlung um eine Vorbehandlung mit Natriumsulfat, auch Kraft-Verfahren genannt, eine Vorbehandlung durch das Ammonia-Fiber-Expansion-Verfahren, auch AFEX-Vorbehandlung genannt, oder eine Vorbehandlung durch Perkolation unter Verwendung von Ammoniak, der recycelt werden kann, auch ARP-Vorbehandlung genannt, ist.

11. Verfahren nach einem der Ansprüche 1 bis 10, wobei der Schritt b) zum Waschen erforderlich ist, wenn es sich bei der alkalischen Vorbehandlung um eine Kraft- oder ARP-Vorbehandlung handelt.

12. Verfahren nach einem der Ansprüche 1 bis 11, wobei der Schritt c) zur enzymatischen Hydrolyse mithilfe der Enzyme vom Typ Cellulasen und/oder Hemicellulasen durchgeführt wird, die von einem Mikroorganismus erzeugt werden, bei dem es sich um einen Pilz handelt, der zu den Gattungen *Trichoderma, Aspergillus, Penicillium* oder *Schizophyllum* gehört, oder um ein anaerobes Bakterium, das zur Gattung *Clostridium* gehört, handelt.

13. Verfahren nach einem der Ansprüche 1 bis 12, wobei der Schritt c) derart durchgeführt wird, dass zwischen 20 und 90 % und vorzugsweise zwischen 30 und 80 % der Zellulose, die in dem vorbehandelten und gewaschenen Brei enthalten ist, in Glukose umgewandelt wird.

14. Verfahren nach einem der Ansprüche 1 bis 13, wobei es sich bei dem am Ende von Schritt e) erhaltenen Alkohol um Ethanol handelt.

15. Verfahren nach einem der Ansprüche 1 bis 13, wobei es sich bei dem am Ende von Schritt e) erhaltenen Lösungsmittel um ein Aceton-Butanol-Ethanol-Gemisch handelt.

16. Verfahren nach einem der Ansprüche 2 bis 11, wobei der Schritt f) zum Abtrennen des Kuchens stromabwärts der Schritte c), d) und/oder e) durchgeführt und gegebenenfalls mit einer Wäsche des Kuchens kombiniert wird.

## Claims

1. Process for the production of alcohols and/or solvents from cellulosic or lignocellulosic biomass that comprises at least the following stages:
a) Alkaline chemical pretreatment of a cellulosic or lignocellulosic substrate;
b) Optionally washing of the pretreated substrate;
c) Enzymatic hydrolysis of the substrate that is pretreated and washed using cellulolytic and/or hemicellulolytic enzymes that produce a hydrolyzate and a water-insoluble residue;
d) Microorganism fermentation of the hydrolyzate that is obtained from stage c) and production of a fermentation must that contains at least one alcohol and/or solvent;
e) Separation/purification of alcohol and/or solvent, and
f) Separation of a cake that contains the insoluble residue,
in which at least a portion (13-1) of the cake (13) that is obtained in stage f) is sent into at least one reactor for regeneration of cellulose, before being recycled downstream from stage a) for alkaline chemical pretreatment, the fraction of the cake that is extracted in stage f) that is sent into the regeneration reactor is mixed with an alkaline solution and then heated to a temperature of between 50 and 150°C.

2. Production process according to Claim 1, in which at least one fraction (13-1) that represents between 20 and 100% of the cake (13) is sent into said regeneration reactor.

3. Process according to Claim 2, in which the fraction (13-1) represents between 75 and 100% of the cake (13).

4. Process according to one of Claims 1 to 3, in which at least one fraction (13-2) that represents between 0 and 80% of the cake is directly discharged without recycling.

5. Process according to Claim 4, in which the fraction (13-2) represents less than 25% of the flow of solid residue (13).

6. Process according to one of Claims 1 to 5, in which the fraction (13-1) is mixed with an alkaline solution in the regeneration reactor and then heated to a temperature of between 70 and 150°C, preferably for a period that varies between 10 minutes and 4 hours.

7. Process according to Claim 6, in which said alkaline solution is sodium sulfate, with the temperature in said reactor being between 70 and 150°C, and preferably the dwell time of between 0.5 and 4 hours.

8. Process according to Claim 6, in which said alkaline solution is gaseous ammonia, with the temperature being between 50 and 100°C, and preferably the dwell time being between 10 and 60 minutes.

9. Process according to Claim 6, in which said alkaline solution is a percolated ammoniacal solution, heated to a temperature of between 80 and 140°C.

10. Process according to one of Claims 1 to 9, in which the pretreatment that is carried out in stage a) is a pretreatment with sodium sulfate, also called a Kraft process, a pretreatment by explosion of the fibers with ammonia, also called AFEX pretreatment, or a percolation pretreatment that uses ammonia with recycling, also called ARP pretreatment.

11. Process according to one of Claims 1 to 10, in which the washing stage b) is necessary if the alkaline pretreatment is a Kraft- or ARP-type pretreatment.

12. Process according to one of Claims 1 to 11, in which stage c) for enzymatic hydrolysis is carried out by means of enzymes such as cellulases and/or hemicellulases that are produced by a microorganism that is a mushroom that belongs to the genera *Trichoderma, Aspergillus, Penicillium* or *Schizophyllum,* or an anaerobic bacteria that belongs to the genus *Clostridium.*

13. Process according to one of Claims 1 to 12, in which stage c) is carried out in such a way that between 20 and 90%, and preferably between 30 and 80%, of the cellulose that is contained in the pretreated and washed paste is converted into glucose.

14. Process according to one of Claims 1 to 13, in which the alcohol that is obtained at the end of stage e) is ethanol.

15. Process according to one of Claims 1 to 13, in which the solvent that is obtained at the end of stage e) is an acetone-butanol-ethanol mixture.

16. Process according to one of Claims 2 to 11, wherein stage f) for separation of the cake is carried out downstream from stages c), d) and/or e) and optionally is coupled to a washing of the cake.
